# EUROPEAN PATENT APPLICATION

(11) **EP 3 772 339 A1**
(43) Date of publication of application: **10.02.2021**
(21) Application number: 19190029.9
(22) Date of filing: 05.08.2019
(51) Int. Cl.: A61B 17/12, A61B 18/14, A61M 1/00

(54) **MEDICAL CLOSURE DEVICE FOR CLOSING THE LEFT ATRIAL APPENDAGE**

(71) Applicant: BIOTRONIK AG, 8180 Bülach (CH)
(72) Inventor: Ulmer, Jens, 8703 Erlenbach (CH)
(74) Representative: Randoll, Sören

(57) **Abstract**

The present invention relates to a medical system (100) for closing the left atrial appendage (1) of a heart of a patient, comprising: a catheter (2), wherein the catheter (2) comprises an inflatable balloon (20) configured to be at least partially inserted into the left atrial appendage (1) to temporarily close the left atrial appendage (1), and wherein the catheter (2) further comprises an inner lumen (3) through which blood can be sucked out of the left atrial appendage (1) to bring the left atrial appendage (1) to a collapsed state, wherein the catheter (2) is further configured to adhere portions of an inside (la) of the left atrial appendage (1) to one another when the left atrial appendage (1) is in a collapsed state.

## Description

The present invention relates to a medical system for closing the left atrial appendage of a heart of a patient. Furthermore, the present invention relates to a corresponding method.

To date, different closure systems for closing the left atrial appendage are known. Some of these systems are based on the targeted release of an implant in the left atrial appendage, in order to close the cavity by means of a self-expandable wire mesh. Furthermore, surgical or minimally invasive procedures are known which separate the left atrial appendage from the heart by ligation.

Furthermore, US 2008/0033241 A1 discloses application of a vacuum to deflate or collapse the left atrial appendage (LAA) cavity and the introduction of a tissue adhesive to maintain the LAA in its collapsed state. Further, US 2016/0192912 A1 describes atrial appendage occlusion devices and methods of using the same that employ the application of light having a desired wavelength range to cure an adhesive present within the cavity. Furthermore, US 2008/0033241 A1 teaches a method and apparatus for sutureless placement of an occluding patch for closing the LAA.

Particularly, systems trying to close the cavity of the LAA with the aid of a self-expanding implant may bear the risk that due to the very variable geometry of the human LAA the cavity cannot be closed completely, or that the implant may dislocate after release. Surgical or minimally invasive procedures show these disadvantages only in a very weakened form, but due to the invasiveness of the procedure may have their disadvantages regarding patient well-being and risk of the intervention itself due to said surgery (minithoractic) .

Based on the above, the problem to be solved by the present invention is to provide an improved medical system for closing the LAA that allows closing the LAA in a more secure fashion and without employing a permanent implant.

This problem is solved by a medical system having the features of claim 1. Preferred embodiments of the present invention are stated in the corresponding sub claims and are described below.

According to claim 1, a medical system for closing the left atrial appendage of a heart of a patient is disclosed, comprising: a catheter, wherein the catheter comprises an inflatable balloon configured to be at least partially inserted into the LAA to temporarily close the LAA, and wherein the catheter further comprises an inner lumen through which blood can be sucked out of the LAA to bring the LAA to a collapsed state, and wherein the catheter is configured to adhere portions of an inside of the LAA to one another when the LAA is in a collapsed state. Particularly, said inside is formed by a surface of an inner wall of the LAA, wherein said surface faces or delimits the cavity formed by the LAA. Further particularly, the catheter can adhere said portions to one another (e.g. temporarily) also as an intermediate material providing e.g. an adhesive layer or electrodes for adhering said portions to one another via ablation (see also below).

Particularly, the solution according to the present invention thus envisions to temporarily close the ostium of the LAA (e.g. with the help of a balloon catheter). Once the entrance is closed, the peripheral cavity and the blood in it are sucked out via the inner lumen of the balloon catheter. The resulting reduced pressure in the cavity of the LAA leads to the collapse of the LAA. Preferably, in a second step, the collapsed inner walls of the cavity are permanently connected to each other. The temporary closure of the ostium is then removed again.

According to an embodiment of the medical system, the catheter comprises a distal end portion extending from a distal end of the balloon to a distal end of the catheter, wherein the distal end portion is configured to be inserted into the LAA.

Furthermore, according to an embodiment of the medical system, the catheter is configured to adhere said portions to one another by discharging an adhesive into the LAA.

Furthermore, according to an embodiment of the medical system, the distal end portion of the catheter comprises a perforated wall surrounding an end section of said inner lumen so that the adhesive can be discharged from the inner lumen through the perforated wall.

Examples of adhesives to be used are: fibrin adhesives; biocompatible adhesives, e.g. based on cyanoacrylates (e.g. n-butyl-2-cyanoacrylate). Furthermore, adhesives based on sclerosing drugs such as those used in varicose vein treatment (sodium tetradecyl sulfate and/or polidocanol) would be conceivable.

Furthermore, according to an embodiment of the medical system, the inner lumen of the catheter forms a light guide, wherein the catheter is configured to pass light via the light guide into the left atrial appendage to cure the adhesive. Here, the adhesive preferably is a light-activatable adhesive, e.g. based on acrylic.

Furthermore, according to an alternative embodiment of the medical system, the catheter is configured to adhere said portions of said inside to one another by applying radio frequency ablation to said portions. Particularly, to this end, the catheter comprises a plurality of electrodes configured to deliver a radio frequency electrical current to said portions of the inside of the left atrial appendage to generate said radio frequency ablation. Further, according to an embodiment, said electrodes protrude from the distal end portion of the catheter.

Furthermore, according to an embodiment, the distal end portion of the catheter comprising the electrodes is connected to an adjacent portion of the catheter through a releasable connection (e.g. a screwed connection), particularly such that the distal end portion can stay in the left atrial appendage while the adjacent, i.e., the remaining portion of the catheter, can be removed from the body of the patient. Preferably, the distal end portion of the catheter is formed out of a biodegradable material so that it does not permanently remain in the LAA.

According to yet another alternative embodiment of the medical system, for adhering said portions of said inside to one another, the balloon comprises an outer side on which an adhesive layer is arranged. Particularly, possible adhesives that can be used for forming the adhesive layer are e.g. described in "Tough adhesives for diverse wet surfaces", J. Li, A. D. Celiz, J. Yang, Q. Yang, I. Wamala, W. Whyte, B. R. Seo, N. V. Vasilyev, J. J. Vlassak, Z. Suo and D. J. Mooney, Science 357 (6349), 378-381.

Furthermore, according to an embodiment of the medical system, the balloon is configured to contact an inside of the LAA in an essentially form fitting manner, so as to attach the adhesive layer to the inside of the left atrial appendage.

According to a further embodiment, the balloon is configured to be deflated to thereby collapse the LAA when the adhesive layer has been attached to an inside of the LAA.

Furthermore, according to an embodiment of the medical system, the catheter is configured to suck blood out of the LAA via the inner lumen upon deflation of the balloon.

Further, according to an embodiment of the medical system, a portion of the catheter comprising the balloon is connected to an adjacent portion of the catheter through a releasable connection (e.g. a screwed connection). Thus, once the balloon attached to the inside of the left atrial appendage through the adhesive layer is deflated, said portion comprising the balloon can be released from the remaining catheter. Preferably, in an embodiment, said portion of the catheter comprising the balloon is formed out of a biodegradable material so that the balloon will remain in the collapsed cavity of the left atrial appendage for a limited amount of time.

Particularly, said material of the portion comprising the balloon can be or comprise a biodegradable polymer (e.g. PLLA, PLGA, PTMC, an alloy comprising magnesium or an alloy/material comprising a combination of the aforementioned materials).

A further aspect of the present invention relates to a method for closing the LAA of a heart of a patient, wherein the method preferably uses a medical system according to the present invention, wherein the method comprises the steps of:
- at least partially inserting the inflatable balloon into the LAA and inflating the balloon to temporarily close the LAA,
- sucking blood out of the LAA through the inner lumen of the catheter to bring the LAA to a collapsed state, and
- adhering portions of an inside of the LAA to one another using the catheter when the LAA is in a collapsed state.

According to an embodiment of the method, the step of adhering portions of said inside of the LAA comprises adhering said portions of the inside of the LAA to one another by discharging an adhesive into the LAA.

According to a further embodiment of the method, the method comprises discharging said adhesive from the inner lumen of the catheter through the perforated wall.

According to a further embodiment of the method, the method comprises passing light via the inner lumen into the LAA to cure the adhesive.

According to an alternative embodiment of the method, the step of adhering portions of said inside of the LAA comprises adhering said portions to one another by applying radio frequency ablation to said portions. Further, according to an embodiment of the method, the method comprises delivering a radio frequency electrical current to said portions of the inside of the LAA to generate said radio frequency ablation.

According to a further embodiment, the method comprises the step of disconnecting the releasable connection between the distal end portion of the catheter that comprises the electrodes and the adjacent portion of the catheter. Particularly, the method comprises the further step of letting the distal end portion of the catheter comprising the electrodes biodegrade in the LAA.

According to yet another alternative embodiment of the method, the step of adhering portions of said inside of the LAA comprises adhering said portions of said inside to one another by attaching the adhesive layer of the balloon to said inside, particularly in a form fitting manner. Particularly, the method further comprises the step of deflating the balloon after attaching the adhesive layer to the inside of the LAA to thereby collapse the LAA.

According to a further embodiment of the method, the method comprises the further step of sucking blood out of the LAA through the inner lumen of the catheter upon deflation of the balloon that is attached to the inside of the LAA through the adhesive layer.

According to a further embodiment, the method comprises the step of disconnecting the releasable connection between the portion of the catheter that comprises the balloon and the adjacent portion of the catheter. Particularly, the method comprises the further step of letting the portion of the catheter comprising the balloon biodegrade in the LAA.

In the following, embodiments, further features and advantages of the present invention shall be described with reference to the Figures, wherein
- Fig. 1: shows a view of the LAA into which a balloon of a catheter of an embodiment of a medical system is inserted, wherein the cavity is emptied via a perforated distal area of an inner lumen of the catheter;
- Fig. 2: shows a side view of Fig. 1;
- Fig. 3: shows the cavity of Figs. 1 and 2 being emptied through the inner lumen of the catheter and at the same time partially filled with adhesive;
- Fig. 4: shows pulling back the catheter shown in Figs. 1 to 3 and spraying of further adhesive into the cavity to close the LAA;
- Fig. 5: shows a further embodiment of a medical system according to the present invention, whereby the cavity is emptied via a perforated distal area of an inner lumen of a catheter of the system, wherein heat is then applied via electrodes in order to glue the inner walls of the cavity together;
- Fig. 6: shows the situation after the inner walls of the LAA have been glued together by said heat generation, wherein the balloon catheter is then detached from the electrode part by means of a releasable connection, so that the distal end portion comprising the electrodes remains in the collapsed LAA and degrades after some time;
- Fig. 7: shows a further embodiment of a medical system according to the present invention comprising a balloon catheter with an adhesive layer arranged on the balloon, wherein the balloon is arranged in the non-collapsed LAA;
- Fig. 8: shows the balloon of Fig. 7 dilated to a positive fit to the inside of the LAA, wherein the remaining cavity is deflated simultaneously via the inner lumen of the catheter;
- Fig. 9: shows establishing a permanent connection between the balloon wall/adhesive layer and the inside of the LAA by means of the positive fit, wherein the catheter shaft is then separated from the balloon part; and
- Fig. 10: shows the collapsed LAA and balloon part after the procedure shown in Figs. 7 to 9.

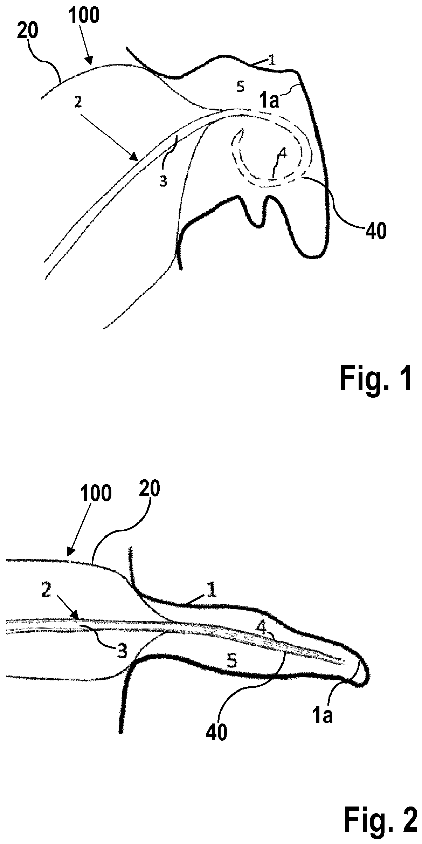

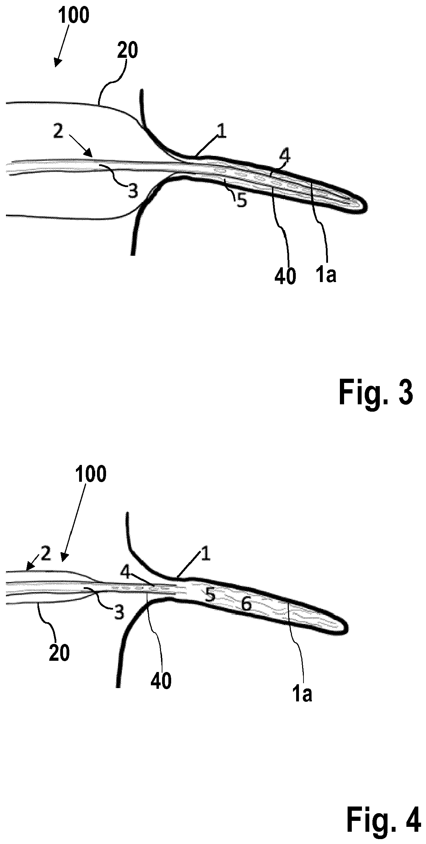

Figs. 1 to 4 show an embodiment of a medical system 100 according to the present invention as well as an embodiment of a method using the system 100 to close a LAA 1 of a heart of a patient.

According to Fig. 1, the system 100 comprises a catheter 2 on which an inflatable balloon 20 is arranged. Using the balloon 20 the LAA 1 is temporarily closed by inserting the latter into the cavity 5 formed by the LAA 1 and inflating the balloon 20 thereafter. Blood remaining in the cavity 1 is then drained through a perforated wall 4 of a distal end portion 40 of the catheter 2 and an inner lumen 3 of the catheter 2. The resulting vacuum causes the cavity 5 to collapse. Subsequently, a biocompatible adhesive 6 is introduced through the inner lumen 3 and simultaneously drained until only adhesive 6 and no blood is removed from the cavity 5. By slowly pulling out the catheter 2 and simultaneously injecting further adhesive 6, the cavity 5 is permanently closed by adhering portions of the inside 1a of the LAA 1 to one another through the adhesive 6.

Examples of adhesives 6 that can be used are: fibrin adhesive, biocompatible adhesives based on cyanoacrylates (e.g. n-butyl-2-cyanoacrylate). Furthermore, a light-activatable adhesive (e.g. based on acrylic) can be used, with the inner lumen 3 of the balloon catheter 2 simultaneously acting as a light guide. Furthermore, adhesives based on sclerosing drugs such as those used in varicose vein treatment (sodium tetradecyl sulfate and/or polidocanol) would be conceivable.

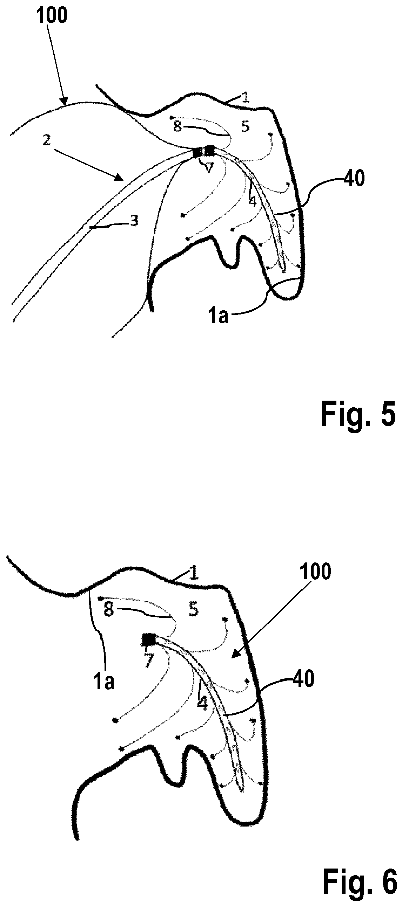

Figs. 5 and 6 show a further embodiment of a medical system according to the present invention. Here, the procedure is performed as described above in conjunction with Figs. 1 to 4, wherein in contrast to adhering portions of the inside 1a of the LAA to one another using an adhesive, the collapsed inside 1a is glued together by RF ablation by means of electrodes 8 that are designed to remain in the cavity 5 of the LAA 1.

Particularly, Fig.5 shows emptying of the cavity 5 of the LAA 1 via a perforated wall 4 of a distal end portion 40 of the catheter 2, which wall 4 surrounds an end section of the inner lumen 3 of the catheter 2 of the system 100. Having emptied the cavity 5, heat is applied via electrodes 8 that protrude from said distal end portion 40 of the catheter 2 in order to glue portions of the inside 1a of the LAA to one another, thus closing the LAA 1.

Fig. 6 schematically shows the situation after the inner walls of the LAA 1 have been glued together by said heat generation, wherein the balloon catheter 2 is then detached from the distal end portion 40 carrying the electrodes 8 by means of a releasable connection 7 so that the distal end portion 40 comprising the electrodes 8 remains in the collapsed LAA 1 and degrades after some time (in case the material of the distal end portion 40 with electrodes 8 is biodegradable).

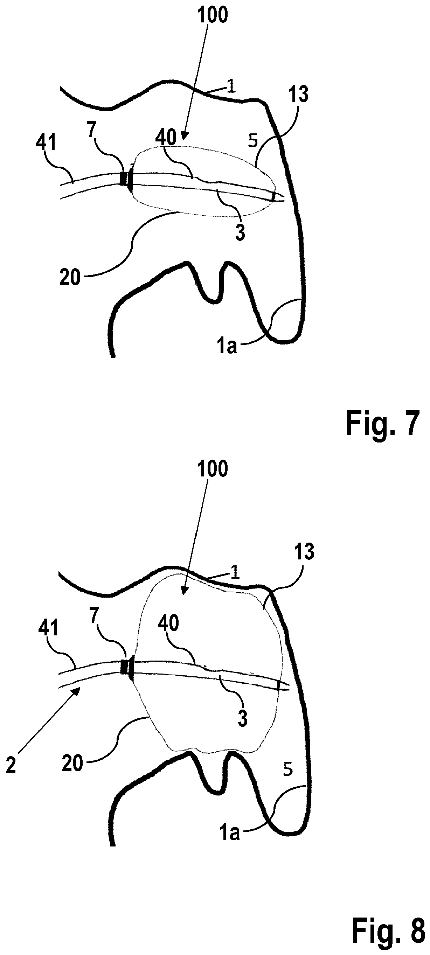

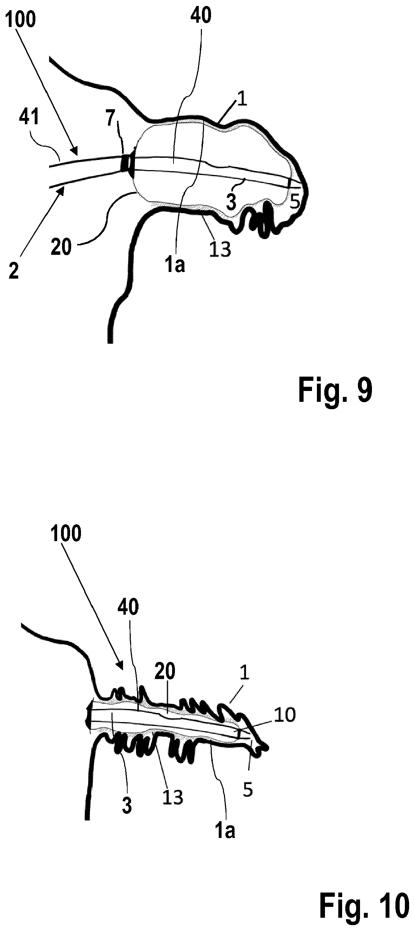

Figs. 7 to 10 show yet another embodiment of a medical system 100 according to the present invention. Here, the balloon 20 of the catheter 2 comprises an adhesive layer 13 on an outside of the balloon 20 that is used to adhere the inside 1a of the LAA to the balloon 20 to ideally create a positive fit between the inside 1a of the LAA 1 and the outside of the balloon 20. After the adhesive contact has been made, which is shown in Fig. 8, the balloon 20 is slowly deflated as shown in Fig. 9, and the cavity 5 of the LAA 1 collapses simultaneously. Via the distal end portion 40 of the catheter, the blood remaining in the cavity 5 is sucked out through the inner lumen 3 of the balloon catheter 2. After having successfully reduced the volume of the LAA 1, the distal end portion 40 comprising the balloon 20 is detached from the rest of the system by disconnecting a releasable (e.g. a screwed) connection 7. Preferably, the distal end portion 40 comprising the balloon 20 consists of a degradable polymer (such as e.g. PLLA, PLGA, PTMC, or a magnesium alloy and/or a combination of the listed materials). Thus, the balloon part 40 of the catheter remaining in the collapsed/closed LAA can biodegrade.

The medical system 100 according to the present invention offers the advantage that occlusion of the LAA 1 is not based on an implantation of a self-expandable implant (e.g. metal framework) or on an invasive (minithoractomy) ligation of the LAA. Therefore, advantageously, dislocation of such an implant can be avoided as no permanent implant is used. Furthermore, the invention has the benefit that leakage can be prevented, as the procedure can be repeated until the entire cavity is closed.

In view of all the foregoing disclosure and while referring to the appended Figures, further embodiments of the present invention are reflected by the following consecutively numbered embodiments:
1. A medical system (100) for closing the left atrial appendage (1) of a heart of a patient, comprising:
   a catheter (2), wherein the catheter (2) comprises an inflatable balloon (20) configured to be at least partially inserted into the left atrial appendage (1) to temporarily close the left atrial appendage (1), and wherein the catheter (2) further comprises an inner lumen (3) through which blood can be sucked out of the left atrial appendage (1) to bring the left atrial appendage (1) to a collapsed state, wherein the catheter (2) is further configured to adhere portions of an inside (1a) of the left atrial appendage (1) to one another when the left atrial appendage (1) is in a collapsed state.
2. The medical system according to embodiment 1, wherein the catheter (2) comprises a distal end portion (40) extending from a distal end of the balloon (20) to a distal end of the catheter (2), wherein the distal end portion (40) is configured to be inserted into the left atrial appendage (1).
3. The medical system according to embodiment 1 or 2, wherein the catheter (2) is configured to adhere said portions of the inside (1a) to one another by discharging an adhesive (6) into the left atrial appendage (1).
4. The medical system according to embodiments 2 and 3, wherein the distal end portion (40) of the catheter (2) comprises a perforated wall (4) surrounding an end section of said inner lumen (3) so that the adhesive (6) can be discharged from the inner lumen (3) through the perforated wall (4).
5. The medical system according to embodiment 3 or 4, wherein the inner lumen (3) of the catheter (2) forms a light guide, wherein the catheter (2) is configured to pass light via the light guide into the left atrial appendage (1) to cure the adhesive (6).
6. The medical system according to embodiment 1 or 2, wherein the catheter (2) is configured to adhere said portions of said inside (1a) to one another by applying radio frequency ablation to said portions of said inside (1a).
7. The medical system according to embodiment 6, wherein the catheter (2) comprises a plurality of electrodes (8) configured to deliver a radio frequency electrical current to said portions of the inside (1a) of the left atrial appendage (1) to generate said radio frequency ablation.
8. The medical system according to embodiments 2 and 7, wherein said electrodes (8) protrude from the distal end portion (40) of the catheter (2).
9. The medical system according to embodiment 2 or 8, wherein the distal end portion (40) of the catheter (2) is connected to an adjacent portion (41) of the catheter (2) via a releasable connection (7).
10. The medical system according to one of the embodiments 2, 8, 9, wherein the distal end portion (40) of the catheter (2) is formed out of a biodegradable material.
11. The medical system according to embodiment 1, wherein for adhering said portions of said inside (1a) of the left atrial appendage (1) to one another, the balloon (20) comprises an outer side on which an adhesive layer (13) is provided.
12. The medical system according to embodiment 11, wherein the balloon (20) is configured to contact the inside (1a) of the left atrial appendage (1) in a form fitting manner to attach the adhesive layer (13) to the inside (1a) of the left atrial appendage (1), and wherein particularly the balloon (20) is configured to be deflated when the adhesive layer (13) is attached to said inside (1a) to thereby collapse the left atrial appendage (1), and wherein particularly the catheter (2) is configured to suck blood out of the left atrial appendage (1) via the inner lumen (3) upon deflation of the balloon (20).
13. The medical system according to one of the embodiments 11 to 12, wherein a portion (40) of the catheter (2) comprising the balloon is connected to an adjacent portion (41) of the catheter (2) via a releasable connection (7).
14. The medical system according to one of the embodiments 11 to 13, wherein said portion (40) of the catheter (2) comprising the balloon (20) is formed out of a biodegradable material.
15. A method for closing the left atrial appendage (1) of a heart of a patient using a medical system (100) according to one of the preceding embodiments, wherein the method comprises the steps of:
   - at least partially inserting the inflatable balloon (20) into the left atrial appendage (1) and inflating the balloon (20) to temporarily close the left atrial appendage (1),
   - sucking blood out of the left atrial appendage (1) through the inner lumen (3) of the catheter (2) to bring the left atrial appendage (1) to a collapsed state, and
   - adhering portions of an inside (1a) of the left atrial appendage (1) to one another using the catheter (2) when the left atrial appendage (1) is in the collapsed state.

## Claims

1. A medical system (100) for closing the left atrial appendage (1) of a heart of a patient, comprising:
a catheter (2), wherein the catheter (2) comprises an inflatable balloon (20) configured to be at least partially inserted into the left atrial appendage (1) to temporarily close the left atrial appendage (1), and wherein the catheter (2) further comprises an inner lumen (3) through which blood can be sucked out of the left atrial appendage (1) to bring the left atrial appendage (1) to a collapsed state, wherein the catheter (2) is further configured to adhere portions of an inside (1a) of the left atrial appendage (1) to one another when the left atrial appendage (1) is in a collapsed state.

2. The medical system according to claim 1, wherein the catheter (2) comprises a distal end portion (40) extending from a distal end of the balloon (20) to a distal end of the catheter (2), wherein the distal end portion (40) is configured to be inserted into the left atrial appendage (1).

3. The medical system according to claim 1 or 2, wherein the catheter (2) is configured to adhere said portions of the inside (1a) to one another by discharging an adhesive (6) into the left atrial appendage (1).

4. The medical system according to claims 2 and 3, wherein the distal end portion (40) of the catheter (2) comprises a perforated wall (4) surrounding an end section of said inner lumen (3) so that the adhesive (6) can be discharged from the inner lumen (3) through the perforated wall (4).

5. The medical system according to claim 3 or 4, wherein the inner lumen (3) of the catheter (2) forms a light guide, wherein the catheter (2) is configured to pass light via the light guide into the left atrial appendage (1) to cure the adhesive (6).

6. The medical system according to claim 1 or 2, wherein the catheter (2) is configured to adhere said portions of said inside (1a) to one another by applying radio frequency ablation to said portions of said inside (1a).

7. The medical system according to claim 6, wherein the catheter (2) comprises a plurality of electrodes (8) configured to deliver a radio frequency electrical current to said portions of the inside (1a) of the left atrial appendage (1) to generate said radio frequency ablation.

8. The medical system according to claims 2 and 7, wherein said electrodes (8) protrude from the distal end portion (40) of the catheter (2).

9. The medical system according to claim 2 or 8, wherein the distal end portion (40) of the catheter (2) is connected to an adjacent portion (41) of the catheter (2) via a releasable connection (7).

10. The medical system according to one of the claims 2, 8, 9, wherein the distal end portion (40) of the catheter (2) is formed out of a biodegradable material.

11. The medical system according to claim 1, wherein for adhering said portions of said inside (1a) of the left atrial appendage (1) to one another, the balloon (20) comprises an outer side on which an adhesive layer (13) is provided.

12. The medical system according to claim 11, wherein the balloon (20) is configured to contact the inside (1a) of the left atrial appendage (1) in a form fitting manner to attach the adhesive layer (13) to the inside (1a) of the left atrial appendage (1), and wherein particularly the balloon (20) is configured to be deflated when the adhesive layer (13) is attached to said inside (1a) to thereby collapse the left atrial appendage (1), and wherein particularly the catheter (2) is configured to suck blood out of the left atrial appendage (1) via the inner lumen (3) upon deflation of the balloon (20).

13. The medical system according to one of the claims 11 to 12, wherein a portion (40) of the catheter (2) comprising the balloon is connected to an adjacent portion (41) of the catheter (2) via a releasable connection (7).

14. The medical system according to one of the claims 11 to 13, wherein said portion (40) of the catheter (2) comprising the balloon (20) is formed out of a biodegradable material.

15. A method for closing the left atrial appendage (1) of a heart of a patient using a medical system (100) according to one of the preceding claims, wherein the method comprises the steps of:
- at least partially inserting the inflatable balloon (20) into the left atrial appendage (1) and inflating the balloon (20) to temporarily close the left atrial appendage (1),
- sucking blood out of the left atrial appendage (1) through the inner lumen (3) of the catheter (2) to bring the left atrial appendage (1) to a collapsed state, and
- adhering portions of an inside (1a) of the left atrial appendage (1) to one another using the catheter (2) when the left atrial appendage (1) is in the collapsed state.
